Europäisches Patentamt

(19) European Patent Office

Office européen des brevets

(11) Publication number : **0 357 725 B1**

## (12) EUROPEAN PATENT SPECIFICATION

(45) Date of publication of patent specification :
**15.04.92 Bulletin 92/16**

(51) Int. Cl.⁵ : **A61K 45/06,** A61K 31/195, A61K 31/135, A61K 37/24

(21) Application number : **89902559.7**

(22) Date of filing : **10.02.89**

(86) International application number :
**PCT/SE89/00057**

(87) International publication number :
**WO 89/07454 24.08.89 Gazette 89/20**

(54) **PHARMACEUTICAL COMPOSITION FOR TREATMENT OF LUNG OBSTRUCTIONS.**

(30) Priority : **11.02.88 SE 8800461**

(43) Date of publication of application :
**14.03.90 Bulletin 90/11**

(45) Publication of the grant of the patent :
**15.04.92 Bulletin 92/16**

(84) Designated Contracting States :
**AT BE CH DE FR GB IT LI LU NL SE**

(56) References cited :
**CH-A- 622 703**
**DE-A- 3 237 253**
**FR-A- 2 357 246**
**US-A- 4 086 363**
**Zeitschrift für die gesamte experimentelle Medizin Volume 98, issued1936 (Berlin), St. Karady, "Uber Thyroxin-und Histaminan- tagonismus", see pages 13-17, especially p.14, fifth paragraph, and p.17**
**Chemical Abstracts, Vol. 52 (1958), abstract No. 8377d, Ormonologia (Turin) 16, 435-46 (1956)**

(56) References cited :
**Chemical Abstracts, Vol. 81 (1974), abstract No. 33568s, Laryngoscope 1974, 84(1), 43-52 (Eng)**
**Chemical Abstracts, Vol. 102 (1985), abstract No. 198460r, Med. Hypothesis 1985, 16(2), 97-131 (Eng)**
**Rote Liste 1986, published 1986, by Bundes- verband der Pharmazeutischen Industrie e.V., Editio Cantor (Aulendorf/Württ.). see Prepa- ration No. 27133 (Asthmastop).**

(73) Proprietor : **SMITH, Ulf Per Gustav**
**Strandragsvägen 8**
**S-434 00 Kungsbacka (SE)**
Proprietor : **WESSLAU, Christian**
**Sjömilsgatan 12**
**S-421 37 Västra Frölunda (SE)**

(72) Inventor : **SMITH, Ulf Per Gustav**
**Strandragsvägen 8**
**S-434 00 Kungsbacka (SE)**
Inventor : **WESSLAU, Christian**
**Sjömilsgatan 12**
**S-421 37 Västra Frölunda (SE)**

(74) Representative : **Inger, Lars Ulf Bosson**
**L + U INGER Patentbyra HB Garvaregatan 12**
**S-262 00 Ängelholm (SE)**

EP 0 357 725 B1

# Description

The present invention relates to the use of beta-adrenergic agonists and thyroid hormone for the manufacture of a pharmaceutical composition having therapeutical and/or prophylactic effect against obstructional conditions in the lungs.

The object of the present invention is to obtain a possibility to improve the therapeutical and prophylactical treatment of obstructional conditions of the lungs, such as asthma and cronical bronchitis by increasing the effect of beta-adrenergic agonists, as well as unspecifically widen the bronchii.

It is previously known to use beta-adrenergic agonists, so called beta-stimulators, and more particularly beta-2-stimulators, in treating asthmatic conditions, whereby a beta-2-stimulator is normally administered locally to the bronchii and, there, by means of its stimulating effect on the beta-receptors present creates a widening of the bronchii and thereby provides for a more easy breathing mechanism.

It is further known that isoprenaline in increasing doses ($10^{-9}$ to $10^{-4}$M) increases the activity of adenylate cyclase in cells of normal as well as hypothyroidic, as well as hyperthyroidic patients.

CH-A-0 622 703 discloses a pharmaceutical composition for the treatment of cellulitis comprising i.a. adrenaline and triiodothyronine or thyroxine.

It has now surpringly been shown possible to be able to increase the effect of adrenergic beta-receptor agonists in the treatment of lung obstructions by means of the present invention which is characterized in that a therapeutically active amount of a beta-adrenergic agonist, as well as a therapeutically active amount of at least one active thyroid hormone are used in the manufacture of pharmaceutical compositions for treating obstructional conditions in the lung.

Further characteristics are evident from the accompanying claims.

Suitable beta-adrenergic agonists are preferably salbutamole, terbutaline, phenotrole, epinephrine, isoprenaline, orciprenaline, and riniterole, as well as other unspecific beta-2-agonists and specific beta-2-agonists. The active compounds are generally used in salt form, such as hydrochloride and sulphate.

Suitable active thyroid hormones are thyroxine, triiodothyronine, diiodothyronine, and other active analogues of thyronine.

The compositions used according to the present invention have turned out to be active preferably in the treatment of asthmatic conditions, chronical bronchitis, and other diseases involving an obstruction of the lungs.

The invention will be described more in detail in the following with reference to some examples however, without being restricted thereto.

## Example 1

Terbutaline sulphate and triiodothyronine were granulated together using ethanol/water together with 1-2% of sorbitan, calculated on the weight of the therapeutically active compounds, as a granulating agent, dried and then ground to a fine powder having a particle size suitable for being administered in the form of an inhalation aerosole for the local treatment of the lungs.

The molar ratio of terbutaline:triiodothyronine was 50:50.

In treatment of an asthmatic patient the daily dose of terbutaline could be decreased by 40 % by weight in acute asthmatic attacks. With a daily maintenance dose used for prophylactic reasons, which was 50 % by weight of a normal terbutaline dose the number of asthmatic attacks could be considerably reduced. This proves that the present composition possesses a therapeutic effect as well as a prophylactic effect.

## Example 2

Salbutamole and thyroxine were treated in the same way as in the Example 1 above for the formulation of an aerosole for local administration to the bronchii.

The molar ratio of salbutamole:thyroxine was 60:40.

In the treatment of an asthmatic patient the same good results were obtained as when using the composition according to Example 1. The therapeutic and prophylactic dose could thus be lowered to the corresponding degree.

By means of the present invention the daily therapeutical dose of beta-adrenergic agonists can thus be lowered with up to 60% when discussing terbutaline.

The ratio of beta-adrenergic stimulator to thyroid hormone is 100:1, preferably 10:1 and down to 1:1. Suitable single doses are 1 to 1000 μg of active compound, suitably 10 to 500 μg. The composition can be administered 1 to 3 times a day depending on the status of the patient.

The active compounds can be administered together with every suitable inert diluent and expedient to the formation of an aerosole which distributed through an inhaler to the bronchii. The compositions can also be produced in the form of a solution intended for injection when a larger acute dose has to be administered to for example an unconscious patient in order to rapidly improve the lung status. In such cases the components are dissolved in the form of water-soluble salts in an isotonic saline solution which is injected, preferably intravenously, for a more rapid effect.

Other types of administration than aerosol powder and injectable solutions may exist, preferably for prophylactic treatment when the composition can be administered in the form of tablets, pills, rectal pills, or granules, coated or uncoated.

## Claims

1. The use of a therapeutically active amount of at least one beta adrenergic agonist and a therapeutically active amount of at least one active thyroid hormone for the manufacture of pharmaceutical compositions for use in the prophylactic and/or therapeutic treatment of obstructional conditions in the lung.

2. The use according to claim 1, whereby the amount of beta adrenergic agonist is reduced by up to 60% in relation to the situation when the agonist is used alone for the same indication.

3. The use according to claim 1, wherein the amount of active thyroid hormone is reduced by up to 60% in relation to the situation when the hormone is used alone.

4. The use according to claims 1 to 3, wherein the beta adrenergic agonist is selected from salbutamole, terbutaline, phenoterole, epinephrine, isoprenaline, orciprenaline, riniterole and other unspecific and specific beta-2-adrenergic agonists.

5. The use according to claims 1 to 3, wherein the active thyroid hormone is selected from thyroxine, triiodothyronine, diiodo-thyronine, and other active thyronine analogues.

6. The use according to claims 1 to 5, wherein the pharmaceutical composition is present as an administration form suitable for inhalation.

7. The use according to claims 1 to 5, wherein the pharmaceutical composition is present as an injectable solution.

8. The use according to claims 1 to 5, wherein the pharmaceutical composition is present as an administration form for peroral use.

9. The use according to claims 1 to 8, wherein the molar ratio between the beta adrenergic agonist and the thyroid hormone is 100:1 - 1:1, preferably 10:1 - 1:1.

10. The use according to claims 1 to 9, wherein the single dose of active components is 1 to 1000 μg, preferably 10 to 500 μg, when administered as an inhalation composition.

## Patentansprüche

1. Verwendung einer therapeutisch aktiven Mengen wenigstens eines beta-adrenergischen Agonisten und einer therapeutisch aktiven Menge wenigstens eines aktiven Schilddrüsenhormons für die Herstellung pharmazeutischer Zusammensetzungen für die Verwendung bei der prophylaktischen und/oder therapeutischen Behandlung von Verstopfungsbedingungen in der Lunge.

2. Verwendung nach Anspruch 1, bei der die Menge an beta-adrenergischem Agonisten um bis zu 60 % in Bezug auf die Situation, wenn der Agonist für die gleiche Indikation allein verwendet wird, vermindert wird.

3. Verwendung nach Anspruch 1, bei der die Menge an aktivem Schilddrüsenhormon um bis zu 60 % in Bezug auf die Situation, wenn das Hormon allein verwendet wird, vermindert wird.

4. Verwendung nach Anspruch 1 bis 3, bei der der beta-adrenergische Agonist unter Salbutamol, Terbutalin, Phenoterol, Epinephrin, Isoprenalin, Orciprenalin, Riniterol und anderen unspezifischen und spezifischen beta-2-adrenergischen Agonisten ausgewählt wird.

5. Verwendung nach den Ansprüchen 1 bis 3, bei der das aktive Schilddrüsenhormon unter Thyroxin, Trijodthyronin, Dijodthyronin und anderen aktiven Thyroninanalogen ausgewählt wird.

6. Verwendung nach den Ansprüchen 1 bis 5, bei der die pharmazeutische Zusammensetzung als eine für Inhalation geeignete Verabreichungsform vorliegt.

7. Verwendung nach den Ansprüchen 1 bis 5, bei der die pharmazeutische Zusammensetzung als eine injizierbare Lösung vorliegt.

8. Verwendung nach den Ansprüchen 1 bis 5, bei der die pharmazeutische Zusammensetzung als eine Verabreichungsform für perorale Verwendung vorliegt.

9. Verwendung nach den Ansprüchen 1 bis 8, bei der das Molverhältnis zwischen beta-adrenergischem Agonisten und dem Schilddrüsenhormon 100 : 1 bis 1 : 1, vorzugsweise 10 : 1 bis 1 : 1 ist.

10. Verwendung nach den Ansprüchen 1 bis 9, bei der die Einzeldosis an aktiven Komponenten 1 bis 100 μg, vorzugsweise 10 bis 500 μg, bei Verabreichung als Inhalations-zusammensetzung ist.

## Revendications

1. Utilisation d'une quantité thérapeutiquement active d'au moins un agoniste bêta-adrénergique et d'une quantité thérapeutiquement active d'au moins une hormone thyroïdienne active dans la fabrication de composés pharmaceutiques utilisés dans le traitement prophylactique et/ou le traitement thérapeutique d'états obstruction des poumons.

2. Utilisation suivant la revendication 1, pour laquelle la quantité de l'agoniste bêta-adrénergique est réduite d'une quantité allant jusque 60 % par rapport au cas dans lequel on utilise l'agoniste seul pour la même indication.

3. Utilisation suivant la revendication 1, pour laquelle la quantité de l'hormone thyroïdienne active est réduite d'une quantité allant jusqu'à 60 % par rapport au cas dans lequel l'hormone est utilisée seule.

4. Utilisation suivant les revendications 1 à 3, pour laquelle l'antagoniste bêta-adrénergique est choisi parmi le salbutamole, la terbutaline, le phénotérole, l'épinéphrine, l'isoprénaline, l'orciprénaline, le rinitérole, et d'autres agonistes bêta-adrénergique non spécifiques et spécifiques.

5. Utilisation suivant les revendications 1 à 3, pour laquelle l'hormone thyroïdienne active est choisie parmi le thyroxine, la triiodothyronine, la diiodothyronine et d'autres analogues actifs de thyronine.

6. Utilisation suivant les revendications 1 à 5, pour laquelle le composé pharmaceutique est utilisé sous une forme d'administration convenant pour une inhalation.

7. Utilisation suivant les revendications 1 à 5, pour laquelle le composé pharmaceutique est utilisé sous forme d'une solution injectable.

8. Utilisation suivant les revendications 1 à 5, pour laquelle le composé pharmaceutique est utilisé sous une forme d'administration destinée à une utilisation par voie orale.

9. Utilisation suivant les revendications 1 à 8, pour laquelle le rapport molaire entre l'agoniste bêta-adrénergique et l'hormone thyroïdienne est de 100/1 à 1/1, de préférence de 10/1 à 1/1.

10. Utilisation suivant les revendications 1 à 9, pour laquelle la dose individuelle des composants actifs est de 1 à 1000 µg, de préférence de 10 à 500 µg, lors d'une administration sous forme d'un composé employé par inhalation.